# EUROPÄISCHE PATENTSCHRIFT

(11) **EP 0 962 218 B1**
(45) Veröffentlichungstag und Bekanntmachung des Hinweises auf die Patenterteilung: **30.11.2005**
(21) Anmeldenummer: 99109689.2
(22) Anmeldetag: 17.05.1999
(51) Int. Cl.: A61K 7/13

(54) **Verfahren zum oxidativen Färben von menschlichen Haaren**
Method for oxidative dying of human hair
Méthode pour teinture oxidative des cheveux humains

(30) Priorität: 05.06.1998 DE 19825133
(43) Veröffentlichungstag der Anmeldung: 08.12.1999
(73) Patentinhaber: KPSS-Kao Professional Salon Services GmbH, 64297 Darmstadt (DE)
(72) Erfinder: Lorenz, Heribert, 64401 Gross-Bieberau (DE)

(56) Entgegenhaltungen:
- EP-A- 0 462 883
- EP-B- 0 282 551
- DE-A- 3 530 732
- DE-A- 4 123 941
- DE-A- 4 219 981

## Beschreibung

Die vorliegende Erfindung betrifft ein neues Verfahren zum oxidativen Färben von menschlichen Kopfhaaren, das nicht nur haarschonender arbeitet als bisher bekannte und übliche Verfahren, sondern auch eine verbesserte Deckkraft und Farbintensität bzw. -stabilität bewirkt.

Die nach wie vor übliche Art der Haarfärbung ist die Färbung mit Oxidationsfarbstoffen, die unmittelbar vor der Anwendung mit Oxidationsmittel-Zusammensetzungen, insbesondere auf Basis von verdünntem Wasserstoffperoxid, vermischt und auf das Haar aufgetragen werden.

Der pH-Wert dieser gebrauchsfertigen Zusammensetzung liegt dabei in der Regel im alkalischen Bereich, insbesondere bei pH-Werten zwischen etwa 9 und etwa 11.

Durch diese alkalische Behandlung wird das Haar, insbesondere bei häufiger Wiederholung des Färbevorganges, in seiner Struktur geschädigt, vor allem, wenn es sich um bereits vorgeschädigtes Haar handelt. Dieses poröse Haar weist nach der Färbung auch eine schlechte Farbbeständigkeit auf.

Es wurde bereits vorgeschlagen, diese Nachteile der bisher üblichen Haarfärbung dadurch zu überwinden, daß, in Abkehr von der oben beschriebenen Praxis, anstelle der alkalisch eingestellten Zusammensetzungen aus Oxidationsfarbstoff und Oxidationsmittel eine analoge Zusammensetzung eingesetzt wird, deren pH-Wert im schwach sauren Bereich zwischen etwa 5,9 und 6,9 liegt.

Derartige Mittel, die z.B. in den DE-OSen Nr. 35 30 270, 36 28 397 und 36 28 398 beschrieben sind, haben sich in der Tat als wesentlich weniger haarschädigend als alkalische Produkte erwiesen.

EP-B-282551 offenbart ein Verfahren zur oxidativen Haarfärbung mittels alkalischer und Saurer Zusammensetzungen, die auf getrennte Zonen der Haarfaser aufgetragen werden.

EP-A-462883 offenbart ein Verfahren zur Haarfärbung mittels Saurer und alkalischer Zusammensetzugen.

DE-A-3530732 und DE-A-4123941 offenbaren Verfahren zur oxidativen Färbung von Haaren auf der Basis alkalischer Zusammensetzungen.

In manchen Fällen, d.h., bei Einstellung spezieller Farbnuancen, läßt allerdings die Intensität und Stabilität der mit diesen verbesserten Zusammensetzungen erzielten Haarfärbungen etwas zu wünschen übrig.

Die Erfindung hat sich die Aufgabe gestellt, diese Nachteile zu überwinden.

Die Lösung dieser Aufgabe besteht in der Anwendung eines neuen Verfahrens nach Anspruch 1 zum oxidativen Färben menschlicher Haare.

Durch die Anwendung dieses Verfahrens wird eine schonende Färbung des Haares, jedoch gleichzeitig auch eine ausgezeichnete Farbintensität erreicht. Die Zeitspanne der Farbeinwirkung kann durch die Applikation von Wärme, vorzugweise 30 bis 50 °C, beispielsweise 40°C, um etwa ein Viertel bis zur Hälfte reduziert werden.

Die in der ersten Behandlungsstufe zum Einsatz gelangenden, alkalischen eingestellten Oxidationsfarbstoffmischungen, die mindestens je eine Entwickler- und je eine Kupplersubstanz sowie ein Oxidationsmittel enthalten, sind an sich bereits bekannt.

Zu diesen gebrauchsfertigen (d.h., das Peroxid enthaltenden) Mischungen, deren pH-Wert zwischen 8 und 11, vorzugsweise 9 und 10, insbesondere bei 9,5 liegt, wird hierzu auf den Stand der Technik, z.B. auf die Monografie von K. Schrader, "Grundlagen und Rezepturen der Kosmetika", 2. Aufl., S. 784-804 (1989), verwiesen; die dort beschriebenen Produkte sind im Rahmen des erfindungsgemäßen Verfahrens ebenso einsetzbar wie die aus dem umfangreichen Stand der Technik bekannten weiteren Entwickler- und Kupplersubstanzen sowie Nuanceure.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2-Hydroxyethylamino)-5-aminotoluol und 1-Amino-4-bis-(2'-hydroxyethyl)-aminobenzol bzw. deren wasserlösliche Salze; beispielhafte Kupplersubstanzen sind Resorcin, 2-Methylresorcin, 4-Chlorresorcin, 2-Amino-4-chlorphenol, 4-(N-methyl)aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 1,4-Diamino-2-chlor-benzol, 4,6-Dichlorresorcin, 1,3-Diaminotoluol, 4-Hydroxy-1,2-methylendioxybenzol, 1,5-Dihydroxy-naphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol und/oder 1-Methoxy-2-amino-4-(2'-hydroxyethylamino)benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbmischungen kann jeweils etwa 0,25 bis etwa 5 Gew.-%, je nach gewünschter Färbung, betragen.

Als Oxidationsmittel werden vor allem verdünnte Wasserstoffperoxid-Lösungen, -Emulsionen oder -Gele eingesetzt, möglich, aber weniger üblich ist auch die Verwendung weiterer Peroxide wie Erdalkaliperoxiden, Harnstoffperoxid, Melaminperoxid, etc. in entsprechenden stöchiometrischen Mengen.

Die Oxidationsfarbstoffzusammensetzungen können als Lösungen, Cremes, Pasten, Gele, Aerosole, etc. Verwendung finden.

Die Einwirkungszeit auf dem Haar liegt bei jeweils fünf bis dreißig Minuten, insbesondere bei jeweils 10 bis 20, beispielsweise jeweils 15 Minuten für die alkalische und angesäuerte Farbmischung. Das erfindungsgemäße Verfahren eignet sich dabei sowohl zum ganzheitlichen, d.h. erstmaligen Färben der Haare als auch zum Nachfärben.

Dabei kann es zweckmäßig sein, wenn die Einwirkung des alkalischen Färbemittels etwas länger dauert als diejenige des sauer eingestellten Mittels.

Die saure, auf das Haar nach Einwirkung des alkalischen Oxidationsfarbstoffvorproduktes aufgebrachte Zusammensetzung weist insbesondere einen pH-Wert zwischen 2 und 6, insbesondere 2,5 und 5, auf und kann eine Lösung oder ein verdicktes Gel sein.

Als Säuren eignen sich organischen Säuren wie Milchsäure, Weinsäure, Citronensäure, Apfelsäure.

Der pH-Wert der auf dem Haar befindlichen Farbmischung nach Aufbringung der Säure liegt dabei vorzugsweise zwischen 5 und 8, insbesondere 6,5 bis 7,5.

In den folgenden Beispielen wird die Erfindung illustriert.

### Beispiel 1

Auf Strähnen gebleichten Menschenhaares wurde eine Färbezusammensetzung, die durch Vermischen von gleichen Gewichtsteilen einer 6%-igen Wasserstoffperoxidlösung und eines Oxidationsfarbstoffvorprodukts der folgenden Zusammensetzung erhalten wurde, und einen pH-Wert von 9,8 aufwies, aufgebracht.

| Trägermasse | |
|---|---|
| Cetylstearylalkohol | 11,00 (Gew.%) |
| Oleth-5 | 5,00 |
| Ölsäure | 2,50 |
| Stearinsäuremonoethanolamid | 2,50 |
| Cocosfettsäuremonoethanolamid | 2,50 |
| Natriumlaurylsulfat | 1,70 |
| Natriumsulfit | 1,00 |
| 1,2-Propandiol | 1,00 |
| Ascorbinsäure | 0,50 |
| Ammoniumchlorid | 0,50 |
| EDTA, Tetranatriumsalz | 0,20 |
| Parfum | 0,40 |
| Weizenproteinhydrolysat | 0,20 |
| Silica | 0,10 |

| Oxidationsfarbstoffmischung | |
|---|---|
| 2,5,6-Triamino-4-hydroxypyrimidinsulfat | 0,01 |
| 2,5-Diaminotoluolsulfat | 0,55 |
| 4-Chlorresorcin | 0,17 |
| Resorcin | 0,05 |
| 3-Aminophenol | 0,03 |
| Wasser | ad 100,00 |

Nach 15-minütiger Einwirkung wurde, ohne Auspülen, eine der ursprünglich eingesetzten Farbmenge entsprechende Menge eines 12,5%-igen wäßrigen Citronensäuregels auf das Haar aufgebracht und gut durchgekämmt, wobei ein pH-Wert von etwa 7 erreicht wurde, und nach weiterer 15-minütiger Einwirkung mit Wasser gut ausgespült und getrocknet.

Es wurde eine starke, intensive, ausdrucksvolle Mittelblondfärbung erhalten, die auch nach fünf Haarwäschen unverändert war.

Strähnen, die in gleicher Weise 30 Minuten nur mit der alkalischen Farbzusammensetzung behandelt worden waren, zeigten demgegenüber eine schwächere Färbung, die nach fünf Haarwäschen deutlich blasser war als die nach dem erfindungsgemäßen Verfahren erzielte. Darüberhinaus war auch der Griff des Haares nach der nur alkalischen Färbung deutlich rauher.

### Beispiel 2

In eine der Zusammensetzung nach Beispiel 1 entsprechende Trägermasse wurde die folgende Oxidationsfarbstoffmischung eingebracht:

| | |
|---|---|
| 2,5,6-Triamino-4-hydroxypyrimidinsulfat | 0,01 (Gew.-%) |
| 2,5-Diaminotoluolsulfat | 0,90 |
| 2-Amino-4-hydroxypyridin | 0,80 |
| 1-Naphthol | 0,17 |
| 3-Aminophenol | 0,10 |
| Resorcin | 0,03 |

Dieses Produkt wurde mit 6 %-iger H₂O₂-Lösung im Gewichtsverhältnis 1:1 vermischt (pH 9,5) und auf Strähnen aus gebleichtem Menschenhaar aufgebracht.

Nach 15-minütiger Einwirkung wurde, ohne Ausspülen, eine der ursprünglich eingesetzten Farbmenge entsprechende Menge eines wäßrigen 15%-igen Weinsäuregels auf das Haar aufgebracht und gut durchgekämmt, wobei ein pH-Wert von etwa 6 erreicht wurde; nach weiterer 15-minütiger Einwirkung wurde mit Wasser ausgewaschen, shampooniert und getrocknet.

Es wurde eine intensive, glänzende Mahagoni-Färbung erhalten, die auch nach fünf Haarwäschen noch stabil war.

Nach Anwendung ausschließlich einer alkalischen Zusammensetzung wies das Haar nicht nur eine weniger intensive Färbung, sondern auch einen rauhen Griff auf.

### Beispiel 3

In eine der Zusammensetzung nach Beispiel 1 entsprechende Trägermasse wurde die folgende Oxidationsfarbstoffmischung eingebracht:

| | |
|---|---|
| 2,5-Diaminotoluolsulfat | 0,80 (Gew.-%) |
| Resorcin | 0,30 |
| 2-Amino-4-hydroxypyridin | 0,06 |
| 3-Aminophenol | 0,03 |

Dieses Produkt wurde mit 6%-iger H₂O₂-Lösung im Gewichtsverhältnis 1:1 gemischt (pH 9,6) und auf Strähnen aus gebleichtem Menschenhaar aufgebracht.

Nach 20-minütiger Einwirkung wurde, ohne Ausspülen, eine der ursprünglichen Farbmenge entsprechende Menge einer 15%-igen wäßrigen Citronensäurelösung auf das Haar aufgebracht und gut durchgekämmt, wobei ein pH-wert von etwa 6 erreicht wurde; nach weiterer zehnminütiger Einwirkung wurde gründlich ausgespült und getrocknet.

Es wurde eine intensive rotbraune Färbung erhalten, die auch nach fünf Haarwäschen noch stabil war, wobei das Haar einen weichen, lockeren Griff aufwies.

## Patentansprüche

1. Verfahren zum oxidativen Färben von menschlichen Haaren, **dadurch gekennzeichnet, daß** zunächst eine alkalische eingestellte, mindestens eine Entwickler- und mindestens eine Kupplersubstanz sowie ein Oxidationsmittel enthaltende Oxidationsfarbstoffmischung und anschließend, nach fünf- bis dreißigminütiger Einwirkung ohne zwischenzeitliches Spülen, eine sauer eingestellte, Zusammensetzung die mindestens eine Säure, ausgewählt aus Citronensäure, Weinsäure, Milchsäure und Äpfelsäure, enthält, auf die Haare aufgebracht, und das Haar nach erfolgter Farbstoffeinwirkung gespült wird.

2. Verfahren nach Anspruch 1, **dadurch gekennzeichnet, daß** die Einwirkungsdauer des Sauer eingestellte Mittels ebenso lang oder kürzer ist als die Einwirkungsdauer des alkalisch eingestellte Mittels.

3. Verfahren nach Anspruch 1 oder 2, **dadurch gekennzeichnet, daß** die Farbstoffeinwirkung bei 30 bis 50 °C erfolgt.

4. Verfahren nach einem oder mehreren der Ansprüche 1 bis 3, **dadurch gekennzeichnet, daß** die alkalisch eingestellte Oxidationsfarbstoffmischung einen pH-Wert von 8 bis 11 aufweist.

5. Verfahren nach einem oder mehreren der Ansprüche 1 bis 4, **dadurch gekennzeichnet, daß** die mindestens eine Säure enthaltende Zusammensetzung einen pH-Wert von 2 bis 6 aufweist.

6. Verfahren nach einem oder mehreren der Ansprüche 1 bis 5, **dadurch gekennzeichnet, daß** der pH-Wert der auf dem Haar befindlichen Färbemischungen nach dem Aufbringen der Säure enthaltenden Zusammensetzung zwischen 5 und 8 liegt.

## Claims

1. Process for the oxidative dyeing of human hair, wherein first an alkaline oxidation dyestuff composition, comprising at least one developing and at least one coupling substance as well as an oxidizing agent is applied to the hair, and subsequently, after five to thirty minutes processing, without rinsing, an acidic composition comprising at least one acid selected from lactic acid, tartaric acid, citric acid, and malic acid is applied, and the hair is rinsed after processing.

2. Process according to claim 1, wherein the processing time of the acidic composition is equal to or shorter than the processing time of the alkaline composition.

3. Process according to claims 1 or 2, wherein the dyestuff composition is processed at 30° to 50° C.

4. Process according to one or more of claims 1 to 3, wherein the alkaline oxidation dyestuff composition has a pH-value of 8 to 11.

5. Process according to one or more of claims 1 to 4, wherein the composition comprising at least one acid has a pH-value of 2 to 6.

6. Process according to one or more of claims 1 to 5, wherein, after application of the composition comprising the acid, the pH-value of the dyeing mixture on the hair ranges between 5 and 8.

## Revendications

1. Procédé pour teindre par oxydation les cheveux humains, **caractérisé en ce qu'**on applique sur les cheveux d'abord un mélange de colorants par oxydation, rendu alcalin, contenant au moins une substance révélatrice et au moins une substance de couplage ainsi qu'un agent d'oxydation, puis, après une action d'environ cinq à trente minutes sans rinçage intermédiaire, une composition rendue acide, qui contient au moins un acide choisi parmi l'acide citrique, l'acide tartrique, l'acide lactique et l'acide malique, et que l'on rince le cheveu après que l'action du colorant ait eu lieu.

2. Procédé selon la revendication 1, **caractérisé en ce que** la durée d'action du produit rendu acide est aussi longue ou plus courte que la durée d'action du produit rendu alcalin.

3. Procédé selon la revendication 1 ou 2, **caractérisé en ce que** l'action du colorant a lieu à une température de 30 à 50°C.

4. Procédé selon l'une ou plusieurs des revendications 1 à 3, **caractérisé en ce que** le mélange de colorants par oxydation rendu alcalin présente une valeur de pH de 8 à 11.

5. Procédé selon l'une ou plusieurs des revendications 1 à 4, **caractérisé en ce que** la composition contenant au moins un acide présente une valeur de pH de 2 à 6.

6. Procédé selon l'une ou plusieurs des revendications 1 à 5, **caractérisé en ce que** la valeur du pH des mélanges de teinture qui se trouvent sur le cheveu après application de la composition contenant un acide est comprise entre 5 et 8.
